# EUROPÄISCHE PATENTSCHRIFT

(11) **EP 1 537 092 B1**
(45) Veröffentlichungstag und Bekanntmachung des Hinweises auf die Patenterteilung: **07.06.2006**
(21) Anmeldenummer: 03790944.7
(22) Anmeldetag: 29.08.2003
(51) Int. Cl.: C07D 301/12

(54) **INTEGRIERTES VERFAHREN ZUR SYNTHESE VON PROPYLENOXID**
INTEGRATED METHOD FOR SYNTHESISING PROPYLENE OXIDE
PROCEDE INTEGRE POUR LA SYNTHESE D'OXYDE DE PROPYLENE

(30) Priorität: 30.08.2002 DE 10240129
(43) Veröffentlichungstag der Anmeldung: 08.06.2005
(73) Patentinhaber: BASF Aktiengesellschaft, 67056 Ludwigshafen (DE)
(72) Erfinder: BENDER, Michael, 67063 Ludwigshafen (DE); ZEHNER, Peter, 67071 Ludwigshafen (DE); MACHHAMMER, Otto, 68163 Mannheim (DE); MÜLLER, Ulrich, 67435 Neustadt (DE); HARTH, Klaus, 67317 Altleiningen (DE); SCHINDLER, Götz-Peter, 68219 Mannheim (DE); JUNICKE, Henrik, 68165 Mannheim (DE)
(74) Vertreter: Hörschler, Wolfram Johannes
(86) Internationale Anmeldenummer: PCT/EP2003/009616
(87) Internationale Veröffentlichungsnummer: WO 2004/020423

(56) Entgegenhaltungen:
- WO-A-00/10961
- WO-A-00/20404
- WO-A-02/085875
- US-A- 5 599 956

## Beschreibung

Die Erfindung betrifft ein integriertes Verfahren zur Synthese von Propylenoxid. Im Rahmen des erfindungsgemäßen Verfahrens werden die Edukte der Propylensynthese über wenigstens die Schritte Propandehydrierung sowie Wasserstoffperoxiddirektsynthese hergestellt und zu Propylenoxid umgesetzt.

Auf dem Produkt Propylenoxid baut sich eine vielgestaltige und sich stark ausweitende Chemie auf. Die weltweite Herstellerkapazität betrug 1985 etwa 2,9 t Mio pro Jahr, 1993 etwa 4,0 Mio. t pro Jahr und ist seither ständig angewachsen. Bisher wird Propylenoxid überwiegend durch das Chlorhydrin-Verfahren und über indirekte Oxidationsverfahren unter Einsatz von Hydroperoxiden hergestellt. Die großen Nachteile dieser beiden Verfahren sind beispielsweise das Abwasser- bzw. Nebenproduktproblem beim Chlorhydrin-Verfahren sowie die Produktion von Cooxidaten in größeren Mengen bei einem Verfahren über die indirekte Oxidation. Diese Probleme führten in den letzten Jahren zu einer regen Entwicklung von alternativen Verfahren auf dem Gebiet der Propylenoxid-Synthese.

Beispielsweise konnte durch die weitestgehende Integration der Chlor-Erzeugung in das Propylenoxid-Synthese-Verfahren über das Chlorhydrin-Verfahren diese wirtschaftlich sinnvoller gestaltet werden.

Auch im Rahmen der Propylenoxid-Synthese durch indirekte Oxidationsverfahren konnte die nachteilig große Menge an Coprodukten durch die Propen-Oxidation beispielsweise mit Percarbonsäuren, welche in einem der Propen-Oxidation vorangehenden Schritt mit Wasserstoffperoxid hergestellt werden, gesenkt werden, wodurch dieses Verfahren an Rentabilität gewann.

Weitere vorteilhafte Entwicklungen auf dem Gebiet der Propylenoxidsynthese werden u.a. in den Schriften DE-A-101 37 543.3, DE-A-101 35 296.4, DE-A-101 05 527.7 und DE-A-100 32 885.7 behandelt.

Dennoch besteht auch aufgrund der vielseitigen Einsetzbarkeit von Propylenoxid, beispielsweise im Rahmen der Polymerisation von Alkoholen, sowie der damit verbundenen stetig steigenden Nachfrage weiterhin der Bedarf nach einem Propylenoxid-Syntheseverfahren, mit welchem es möglich ist, die gesamte Synthese von Propylenoxid, d.h. angefangen von der Bereitstellung der Edukte bis hin zum Recycling der neben Propylenoxid anfallenden Produkte, noch wirtschaftlicher und damit wettbewerbsfähiger zu gestalten.

Demgemäß lag der Erfindung die Aufgabe zu Grunde, ein weiteres Verfahren zur integrierten Synthese von Propylenoxid bereitzustellen.

Somit betrifft die vorliegende Erfindung ein erweitertes integriertes Verfahren zur Synthese von Propylenoxid, dadurch gekennzeichnet, dass das Verfahren wenigstens die folgenden Schritte umfasst:
(a) Propandehydrierung unter Erhalt eines Teilstroms T (0), welcher wenigstens Propan, Propen und Wasserstoff aufweist;
(b) Auftrennung des Teilstroms T (0) unter Erhalt wenigstens eines gasförmigen wasserstoffreichen Teilstroms T (2) und eines Teilstroms T (1), welcher wenigstens Propen und Propan aufweist;
(c) Auftrennung des Teilstroms T (1) unter Erhalt wenigstens eines propanreichen Teilstroms T (5) und wenigstens eines propenreichen Teilstrom T (3);
(d) Auftrennung des Teilstroms T (5) in wenigstens die Teilströme T (5a) und T (5b);
(e) Wasserstoffperoxidsynthese unter Einsatz des Teilstroms T (2) welcher wenigstens mit dem Teilstrom T (5a) vereint wird, wobei ein wasserstoffperoxidreicher Teilstrom T (4) und ein gasförmiger Teilstrom T (6a) erhalten wird;
(f) Rückführung des Teilstroms T (6a) in Schritt (a);
(g) Umsetzung des wenigstens einen Teilstroms T (3) mit Teilstrom T (4) unter Erhalt von Propylenoxid.

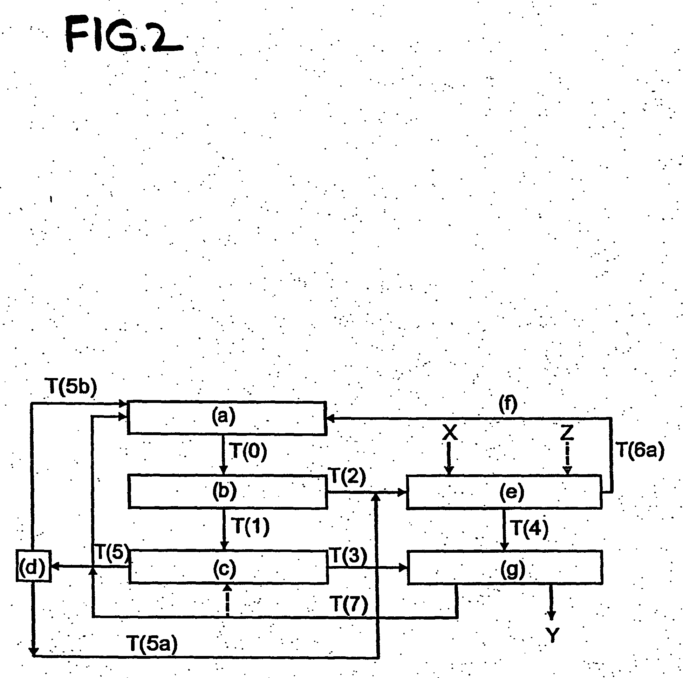

Eine bevorzugte Ausführungsform der Verfahrensführung des erweiterten integrierten Verfahrens zur Synthese von Propylenoxid, d.h. der wesentlichen, das Verfahren umfassenden Schritte ist schematisch in Figur 2 (Fig.2) dargestellt.

Ein alternatives Verfahren, das nicht erfindungsgemaß ist, ist ein integriertes Verfahren zur Synthese von Propylenoxid, welches wenigstens die folgenden Schritte umfasst:
(i) Propandehydrierung unter Erhalt eines Teilstroms T (0), welcher wenigstens Propan, Propen und Wasserstoff aufweist;
(ii) Auftrennung des Teilstroms T (0) unter Erhalt wenigstens eines gasförmigen wasserstoffreichen Teilstroms T (2) und eines Teilstroms T (1), welcher wenigstens Propen und Propan aufweist;
(iii) Wasserstoffperoxidsynthese unter Einsatz des Teilstroms T (2), wobei ein wasserstoffperoxidreicher Teilstrom T (4) und ein gasförmiger Teilstrom T (6) erhalten wird;
(iv) Auftrennung des Teilstroms T (1) unter Erhalt wenigstens eines propanreichen Teilstroms T (5) und wenigstens eines propenreichen Teilstroms T (3);
(v) Umsetzung des wenigstens einen Teilstroms T (3) mit Teilstrom T (4) unter Erhalt von Propylenoxid.

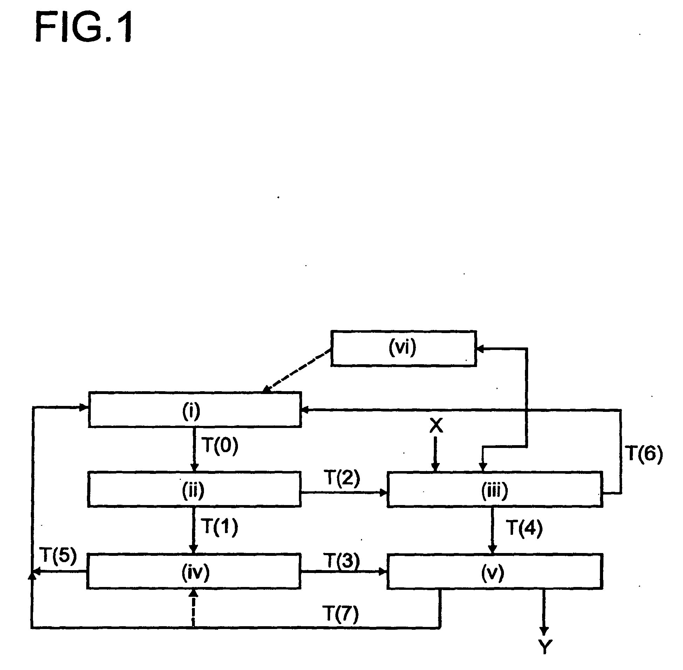

Diese Verfahrensführung, d.h. der wesentlichen, das Verfahren umfassenden Schritte ist schematisch in Figur 1 (Fig.1) dargestellt.

Schritt (i) des alternativen Verfahrens bzw. Schritt (a) der erfindungsgemäßen Verfahren umfasst die Propandehydrierung unter Erhalt eines Teilstroms T (0), welcher wenigstens Propan, Propen und Wasserstoff aufweist.

Die in Rede stehende Propandehydrierung kann im Rahmen des erfindungsgemäßen Verfahrens grundsätzlich durch alle dem Fachmann für die Dehydrierung von Propan bekannten Verfahrensweisen, wie beispielsweise das Steam Cracking oder katalytische Crackverfahren sowie insbesondere auch die katalytische Dehydrierung unter Abwesenheit oder Beisein von Sauerstoff bzw. Sauerstoff enthaltenden Gemischen durchgeführt werden.

Um bei der Propandehydrierung, bezogen auf einen einmaligen Durchgang, wirtschaftlich sinnvolle Umsätze zu erreichen, ist es grundsätzlich notwendig, bei relativ hohen Reaktionstemperaturen zu arbeiten. Diese betragen in der Regel 300 bis 700 °C.

Da die Dehydrierung, d.h. die Spaltung einer C-H Bindung in der Regel gegenüber dem Cracking, d.h. der Spaltung einer C-C Bindung, kinetisch benachteiligt ist, erfolgt sie bevorzugt an für die Dehydrierung selektiven Katalysatoren. Diese sind üblicherweise derart beschaffen, dass mit ihnen unter Ausschluss von Sauerstoff in dem oben aufgeführten Temperaturbereich eine gute Ausbeute an Dehydrierungsprodukten erhalten wird. In der Regel beträgt die Propylenausbeute bei einer Propan-Belastung der Katalysatoren von beispielsweise 1000 h⁻¹ (d.h. Normliter Propan pro Liter Katalysator und Stunde) wenigstens 30 mol.-%, bezogen auf das eingesetzte Propan in einem einmaligen Durchgang. Nebenprodukte wie Methan, Ethylen und Ethan entstehen nur in untergeordneten Mengen.

Da die Propandehydrierung unter Volumenzunahme verläuft, kann der Umsatz grundsätzlich durch Erniedrigung des Partialdrucks der Produkte gesteigert werden. Dies kann in einfacher Weise beispielsweise durch die Durchführung der Dehydrierung bei vermindertem Druck und/oder der Zumischung von im wesentlichen inerten Verdünnungsgasen erreicht werden. Im Rahmen der vorliegenden Erfindung stellt Wasserdampf ein solches bevorzugt verwendetes inertes Verdünnungsgas dar. Weitere für die Propandehydrierung geeignete Verdünnungsgase stellen beispielsweise CO₂, N₂ und Edelgase wie He, Ne und Ar dar.

Eine Verdünnung mit Wasserdampf bedingt als weiteren Vorteil in der Regel ein vermindertes Verkoken des verwendeten Katalysators, da der Wasserdampf mit sich gegebenenfalls bildendem Koks nach dem Prinzip der Kohlevergasung reagiert. Ferner lässt sich Wasserdampf leicht teilweise oder vollständig aus dem Produktgemisch abtrennen. Demgemäß ist es im Rahmen der vorliegenden Erfindung möglich, beim Einsatz von Wasserdampf als Verdünnungsmittel im Rahmen der Propandehydrierung diesen aus dem Produktstrom T (0) beispielsweise durch Kondensation abzutrennen.

Für die Propandehydrierung in Schritt (i) bzw. Schritt (a) können grundsätzlich alle dem Fachmann für diesen Zweck bekannten Dehydrierungskatalysatoren eingesetzt werden. Beispiele sind etwa Katalysatoren oxidischer Natur, welche Chromoxid und/oder Aluminiumoxid aufweisen, oder solche, welche aus wenigstens einem auf wenigstens einem in der Regel oxidischen Träger abgeschiedenen weitgehend edlen Metall, z.B. Platin, bestehen.

Unter anderem können im Rahmen der vorliegenden Erfindung die in folgenden Druckschriften beschriebenen Dehydrierungskatalysatoren eingesetzt werden: WO 99/46039, US 4,788,371, EP-A 705 136, WO 99/29420, US 5,220,091, US 5, 430, 220, US 5,877,369, EP-A 117 146, DE 199 37 106, DE 199 37 105, sowie DE 199 37 107.

Insbesondere können auch die in den Beispielen der DE 199 37 107 beschriebenen Dehydrierungskatalysatoren eingesetzt werden. Bei diesen handelt es sich um Dehydrierungskatalysatoren, welche 10 bis 99,9 Gew.-% Zirkondioxid, 0 bis 60 Gew.-% Aluminiumoxid, Siliziumdioxid und/oder Titandioxid sowie 0,1 bis 10 Gew.-% wenigstens eines Elementes der ersten oder zweiten Hauptgruppe, der dritten oder achten Nebengruppe des Periodensystems der Elemente, Lanthan und/oder Zinn enthalten, mit der Maßgabe, dass die Summe der Gewichtsprozente 100 ergibt.

Zur Durchführung der Propandehydrierung im Rahmen der vorliegenden Erfindung sind grundsätzlich alle dem Fachmann für diesen Zweck bekannten Reaktortypen und Verfahrensführungen einsetzbar, beispielsweise auch die, die in den im vorhergehenden Abschnitt im Rahmen der Dehydrierungskatalysatoren zitierten Druckschriften beschrieben sind.

Beispielsweise ist es möglich, das eingesetzte Propan im Rahmen der Propandehydrierung, wie auch in US 3,798,283 beschrieben, im Beisein von molekularem Sauerstoff bei erhöhter Temperatur homogen zu Propen zu oxidieren. Als Sauerstoffquelle kommen im Rahmen der Erfindung dabei sowohl reiner Sauerstoff als auch Gemische aus Sauerstoff und Inertgas in Betracht.

Auch die in DE 195 30 454 beschriebene Verfahrensweise der heterogen katalysierten Oxidehydrierung von Propan zu Propen kann im Rahmen der Propandehydrierung durchgeführt werden. Dabei wird das Propan in einem mit Katalysator gefüllten Festbett- oder Wirbelschichtreaktor unter Beisein von Luft oder einem Sauerstoff enthaltenden Gemisch zu Propen umgesetzt.

Im Rahmen der Erfindung ist es weiterhin möglich, analog zu dem in DE 198 37 517 beschrieben Verfahren Propan durch homogen und/oder heterogen katalysierte Oxidehydrierung mit molekularem Sauerstoff in Propen zu überführen.

Grundsätzlich können alle im Rahmen der Erfindung einsetzbaren Oxidehydrierungsverfahren in wenigstens einem, mit katalytisch aktiver Substanz gefüllten Reaktionsbehälter, beispielsweise einem Festbett- oder einem Wirbelschichtbettreaktor, durchgeführt werden. In diesem wird das Propan an der jeweilig eingesetzten katalytisch aktiven Substanz über dem Fachmann bekannte Reaktionsschritte zu Propen umgesetzt.

Eine weitere Möglichkeit zur Propandehydrierung im Rahmen des erfindungsgemäßen Verfahrens stellen das Oleflex^{™}-Verfahren oder dazu ähnliche Verfahrensführungen dar. Im Rahmen dieses Verfahrens wird das eingesetzte Propan, vermischt mit reinem oder recyceltem Wasserstoff, in wenigstens einem Reaktor umfassend, wenigstens ein geeignetes Katalysatorbett, zu Propen umgesetzt.

Grundsätzlich kann Propan in Gegenwart eines Dehydrierungskatalysators teilweise oder nahezu vollständig zu Propen dehydriert werden. Bei der teilweisen Dehydrierung wird ein Produktgasgemisch gebildet, welches neben nicht umgesetztem Propan und dem gebildeten Propen Nebenbestandteile wie Wasserstoff, Wasser, weitere Crack-Produkte des Propans, CO und CO₂ enthält. Die Propandehydrierung kann mit einem oder ohne ein sauerstoffhaltigen Gas als Co-Feed durchgeführt werden.

Die partielle heterogen katalysierte Dehydrierung von Propan verläuft in der Regel endotherm, d.h. für die Einstellung der erforderlichen Reaktionstemperatur benötigte Wärme/Energie wird dem Reaktionsgas vorab und/oder während der katalytischen Dehydrierung zugeführt.

Aufgrund der für die Propandehydrierung benötigten relativ hohen Reaktionstemperatur kann es zur Bildung geringer Mengen an schwersiedenden hochmolekularen organischen Verbindungen, unter Umständen auch Kohlenstoffen, kommen, welche sich eventuell auf der Katalysatoroberfläche abscheiden. Um diese negative Begleiterscheinung weitgehend zu minimieren bzw. zu vermeiden, kann im Rahmen der vorliegenden Erfindung das Edukt Propan mit Wasserstoff verdünnt werden, wobei der eventuell entstandene Kohlenstoff nach dem Prinzip der Kohlehydrierung weitgehend eliminiert werden kann.

Eine ausführliche Beschreibung von für die Propandehydrierung im Rahmen der vorliegenden Erfindung grundsätzlich geeigneten Reaktortypen und -fahrweisen enthält "Catalytica® Studies Division, Oxydative Dehydrogenation and Alternative Dehydrogenation Processes, Study No. 4192 OD, 1993, 430, Ferguson Drive, Mountain View, California, 94043-5272, U.S.A.".

Eine im Rahmen der vorliegenden Erfindung für das Verfahren in Schritt (a) geeignete Reaktorform ist der Festbettrohr- oder Rohrbündelreaktor. Bei diesem befindet sich der Katalysator als Festbett in einem Reaktionsrohr oder in einem Bündel von Reaktionsrohren. Die Propandehydrierung kann unter Abwesenheit von Sauerstoff oder bei Verwendung geeigneter Katalysatorformulierungen auch unter Zuführung von Sauerstoff als Co-Feed durchgeführt werden. Die Reaktionsrohre können dadurch beheizt werden, dass in den die Reaktionsrohre umgebenden Raum ein Gas, beispielsweise ein Kohlenwasserstoff wie Methan, verbrannt wird.

Im Rahmen des alternativen Verfahrens zur Synthese von Propylenoxid kann der aus Schritt (iii) hervorgehende gasförmige Teilstrom T (6) ganz oder teilweise zur Energiegewinnung verbrannt werden und die Energie innerhalb der Propandehydrierung beispielsweise zur indirekten Beheizung der in Rede stehenden Reaktionsrohre eingesetzt werden.

Vorteilhaft ist es hierbei, diese indirekte Form der Aufheizung lediglich auf den ersten ca. 20 bis 30 % der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen.

Da der Katalysator im Allgemeinen in Abhängigkeit von Gasbelastung und Umsatz mehr oder weniger-rasch durch Verkokung desaktiviert, ist es von Vorteil, ihn in regelmäßigen Abständen zu regenerieren. Zur Regenerierung kann dabei der aus Schritt (iii) hervorgehende Teilstrom T (6) verwendet werden. Weitere Regenerierungsverfahren, die eingesetzt werden können, sind in WO 98/55430 und dem darin zitierten Stand der Technik beschrieben.

In einer weiteren Ausführungsform der vorliegenden Erfindung kann die Propandehydrierung in einem Wanderbett-Reaktor durchgeführt werden. Das Katalysator-Wanderbett kann beispielsweise in einem Radialstromreaktor untergebracht sein. In diesem bewegt sich der Katalysator langsam von oben nach unten, während das Reaktionsgasgemisch radial strömt. Da die Reaktoren bei diesem Verfahren quasi adiabat betrieben werden, ist es zweckmäßig, mehrere Reaktoren hintereinander geschaltet zu betreiben.

Vor jedem Reaktor kann das Eintrittsgemisch durch indirekte Beheizung auf die erforderliche Reaktionstemperatur aufgeheizt werden. Ebenso ist es möglich, das Eintrittsgasgemisch in jedem Reaktor durch Verbrennung von Wasserstoff in Gegenwart von zugeführtem Sauerstoff auf die erforderliche Reaktionstemperatur aufzuheizen (autotherme Fahrweise). Sowohl im Rahmen des alternativen integrierten als auch erweiterten erfindungsgemäßen integrierten Verfahrens zur Synthese von Propylenoxid ist es vorteilhaft, den aus Schritt (iii) hervorgehenden Teilstrom T (6) bzw. T (6a) ganz oder teilweise zur Beheizung des Eintrittsgasgemisches bzw. im Rahmen der Propandehydrierung des Schrittes (a) zur Unterstützung einer autothermen Verfahrensweise zu verwenden.

Weiterhin lassen sich durch die Verwendung mehrerer Reaktoren große Unterschiede der Temperatur des Reaktionsgasgemisches zwischen Reaktoreingang und Reaktorausgang vermeiden und hohe Gesamtumsätze erzielen. Wenn das Katalysatorbett den WanderbettReaktor verlassen hat, wird es der Regenerierung zugeführt und anschließend wieder verwendet. Die Regenerierung des Katalysators im Wanderbettreaktor erfolgt in der Regel auf kontinuierliche Weise. Dabei wird der verbrauchte Katalysator ganz oder teilweise am Ende eines jeden Reaktors, insbesondere am Ende des letzten Reaktors, ausgetragen und wenigstens einem sich anschließenden Regenerierungsschritt zugeführt. Nach der Regeneration wird der Katalysator am Anfang eines jeden Reaktors, insbesondere am Anfang des ersten Reaktors, wieder zugeführt.

Die heterogen katalysierte, im Wirbelbett durchgeführte Propan-Dehydrierung, deren Verfahrensweise in "Chem. Eng. Sci. 1992 b 47 (9-11), 2313" beschrieben wird, stellt ebenfalls eine mögliche Vorgehensweise zur Propandehydrierung im Rahmen der vorliegenden Erfindung dar. Im Rahmen dieses Verfahrens muss das Propan nicht verdünnt werden. Zweckmäßigerweise werden bei der Dehydrierung zwei Wirbelbetten nebeneinander betrieben, von denen sich eines in der Regel im Zustand der Regenerierung befindet. Die Regenerierung der Wirbelbetten kann im Rahmen des alternativen integrierten Verfahrens zur Synthese von Propylenoxid durch die Verwertung des aus Schritt (iii) hervorgehenden Teilstroms T (6) vorgenommen werden. Die für die Dehydrierung erforderliche Wärme wird dabei in das Reaktionssystem eingebracht, in dem der Dehydrierungskatalysator auf die Reaktionstemperatur vorerhitzt wird. Auch das Vorerhitzen kann durch den ganz oder teilweise zur Energiegewinnung verbrannten Teilstrom T (6) aus Schritt (iii) erfolgen.

Auch ist es im Rahmen einer weiteren Ausführungsform der vorliegenden Erfindung möglich, durch die Zumischung eines Sauerstoff enthaltenden Co-Feeds auf die Vorerhitzer oder Zwischenerhitzer bzw. die indirekte Erhitzung über die Reaktorflächen zu verzichten und die benötigte Wärme ganz oder teilweise direkt im Reaktorsystem durch Verbrennung von Wasserstoff und/oder Kohlenwasserstoffen in Gegenwart von molekularem Sauerstoff zu erzeugen. Demgemäß wird der aus Schritt (e) des erfindungsgemäßen erweiterten integrierten Verfahrens zur Synthese von Propylenoxid hervorgehende Teilstrom T (6a) in einem weiteren Schritt (f) in den die Propandehydrierung umfassenden Schritt (a) zurückgeführt und somit eine weitgehend autotherme Fahrweise der Propandehydrierung ermöglicht. Gegebenenfalls kann zusätzlich ein Wasserstoff enthaltender Co-Feed zugemischt werden.

Auch die Durchführung der Propan-Dehydrierung in einem Hordenreaktor stellt eine mögliche Vorgehensweise im Rahmen der Propandehydrierung der vorliegenden Erfindung dar. Ein Hordenreaktor enthält ein oder mehrere aufeinanderfolgende Katalysatorbetten, welche vorzugsweise radial oder axial von Reaktionsgas durchströmt werden. Im allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von zentrisch ineinander gestellten zylindrischen Gitterrosten angeordnet. Ein Schachtofenreaktor entspricht einer Horde. Bei einer Fahrweise ohne Sauerstoff als Co-Feed wird das Reaktionsgasgemisch im Hordenreaktor auf seinem Weg von einem Katalysatorbett zum nächsten Katalysatorbett einer Zwischenerhitzung unterworfen, beispielsweise durch Überleiten über mit heißen Gasen erhitzte Wärmeaustauscherflächen oder durch Durchleiten durch mit heißen Brenngasen beheizte Rohre.

Bei einer Fahrweise mit Sauerstoff wird je nach verwendetem Dehydrierungskatalysator eine begrenzte Menge der im Reaktionsgas enthaltenen Kohlenwasserstoffe, gegebenenfalls auch bereits auf der Katalysatoroberfläche abgeschiedenen Kohlenstoffen und/oder von im Verlauf der Propandehydrierung gebildetem und/oder dem Reaktionsgas zugesetztem Wasserstoff verbrannt. Die dabei freigesetzte Reaktionswärme ermöglicht es, dass die Propandehydrierung auch autotherm betrieben werden kann.

Auch ist es in einer weiteren Ausführungsform des erfindungsgemäßen Verfahrens möglich, Schritt (a), die Propandehydrierung, autotherm durchzuführen. Hierzu wird dem Reaktionsgemisch der Propan-Dehydrierung in mindestens einer Reaktionszone zusätzlich ein Sauerstoff enthaltendes Gas zugemischt und der in dem Reaktionsgasgemisch enthaltene Wasserstoff verbrannt, wodurch zumindest ein Teil der benötigten Dehydrierwärme in der mindestens einen Reaktionszone direkt im Reaktionsgemisch erzeugt wird.

Bevorzugt wird die Propandehydrierung entsprechend der in DE 102 11 275.4 beschriebenen Kreisfahrweise betrieben.

Im Rahmen des erfindungsgemäßen erweiterten integrierten Verfahrens zur Synthese von Propylenoxid wird zur Durchführung einer autothermen Fahrweise des Propandehydrierungsschritts (a) der in Schritt (e) erhaltene gasförmige, Wasserstoff aufweisende Teilstrom T (6a) ganz oder teilweise in Schritt (a) zurückgeführt und verbrannt.

Durch die Regelung der Menge an mittels Teilstrom T (6a) zugesetztem Sauerstoff ist es möglich, die Reaktionstemperatur innerhalb von Schritt (a) zu steuern. Gleichzeitig ist es möglich, durch die Regelung der Menge an mittels Teilstrom T (6a) zugesetztem Wasserstoff die Selektivität der Propandehydrierung innerhalb des Schrittes (a) zu steuern.

Durch die Menge des dem Reaktionsgasgemisch zugesetzten sauerstoffhaltigen Gases wird die für die Dehydrierung des Propans zu Propen benötigte Wärmemenge, welche durch die Verbrennung des im Reaktionsgasgemisch vorhandenen Wasserstoffs und gegebenenfalls von im Reaktionsgasgemisch vorliegenden Kohlenwasserstoffen und/oder von in Form von Koks vorliegendem Kohlenstoff erzeugt wird, geregelt.

Zusätzlich zugeführter Sauerstoff kann dabei entweder als molekularer Sauerstoff oder als sauerstoffhaltiges Gas, u. a. auch im Gemisch mit Inertgasen, eingesetzt werden.

In einer bevorzugten Ausführungsform der Erfindung wird in diesem Zusammenhang mit zusätzlich zugeführtem molekularem Sauerstoff gearbeitet.

Im Rahmen einer weiteren bevorzugten Ausführungsform der vorliegenden Erfindung ist das in Rede stehende sauerstoffhaltige Gas der aus der Wasserstoffperoxidsynthese in Schritt (e) hervorgehende Teilstrom T (6a).

Die Inertgase und die resultierenden Verbrennungsgase wirken im allgemeinen zusätzlich verdünnend und fördern damit die heterogen katalysierte Dehydrierung. Der zur Wärmeerzeugung verbrannte Wasserstoff kann der bei der Kohlenwasserstoff-Dehydrierung gebildete Wasserstoff sowie der dem Reaktionsgasgemisch zusätzlich zugesetzte Wasserstoff sein, beispielsweise der im erfindungsgemäßen erweiterten integrierten Verfahren zur Synthese von Propylenoxid aus Schritt (e) stammende, Wasserstoff aufweisende Teilstrom T (6a).

Grundsätzlich wird soviel Wasserstoff zugesetzt, dass das Mol-Verhältnis H₂/O₂ im Reaktionsgasgemisch unmittelbar nach der Einspeisung 0 bis 10 mol/mol beträgt. Dies gilt bei mehrstufigen Reaktoren auch für die Zwischeneinspeisung von Wasserstoff und Sauerstoff. Die in Rede stehende Wasserstoffverbrennung erfolgt katalytisch, wobei der eingesetzte Dehydrierungskatalysator im allgemeinen auch die Verbrennung der Kohlenwasserstoffe und die Verbrennung von Wasserstoff mit Sauerstoff katalysiert, so dass grundsätzlich kein von diesem verschiedener spezieller Oxidationskatalysator erforderlich ist. Selbstverständlich kann jedoch auch in Gegenwart eines oder mehrerer Oxidationskatalysatoren gearbeitet werden. Diese katalysieren selektiv die Verbrennung von Wasserstoff zu Sauerstoff in Gegenwart von Kohlenwasserstoffen. Die Verbrennung der Kohlenwasserstoffe mit Sauerstoff zu CO, CO₂ und H₂O läuft dadurch nur in untergeordnetem Maße ab, was sich deutlich positiv auf die erzielte Selektivität für die Bildung von Propen auswirkt. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff in Gegenwart von Kohlenwasserstoffen katalysiert, an den Stellen angeordnet, an denen höhere Sauerstoffpartialdrücke herrschen als an anderen Stellen des Reaktors, insbesondere in der Nähe der Einspeisungsstelle für das sauerstoffhaltige Gas. Die Einspeisung von sauerstoffhaltigem Gas und/oder Wasserstoff kann an einer oder mehreren Stellen des Reaktors erfolgen.

Ein bevorzugter Katalysator, welcher selektiv die Verwendung von Wasserstoff katalysiert, enthält in der Regel Oxide oder Phosphate, ausgewählt aus der Gruppe bestehend aus den Oxiden oder Phosphaten von Germanium, Zinn, Blei, Arsen, Antimon und Bismut.

Ein weiter bevorzugter Katalysator, der die Verbrennung von Wasserstoff katalysiert, enthält wenigstens ein Edelmetall der VIII. Nebengruppe des Periodensystems der Elemente. Beispiele für derartige Katalysatoren sind u.a. in folgenden Schriften beschrieben: US-A 4,788,371, US-A 4,886,928, US-A 5,430,209, US-A 5,530,171, US-A 5,527,979 sowie US-A 5,563,314.

Bevorzugt wird die Propan-Dehydrierung in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit des Katalysators erhöht werden kann. Dabei werden die organischen Ablagerungen in Kohlenmonoxid und Kohlendioxid umgewandelt.

Im Rahmen der vorliegenden Erfindung wird die Propandehydrierung bevorzugt im Hordenverfahren durchgeführt, wobei im Rahmen des erweiterten integrierten Verfahrens zur Synthese von Propylenoxid durch die Verbrennung des durch den Teilstrom T (6a) zugeführten Wasserstoffs eine weitgehend autotherme und dadurch kostengünstige Verfahrensweise ermöglicht wird.

Im Rahmen der vorliegenden Erfindung ist die Qualität des eingesetzten Propans grundsätzlich unkritisch. So kann es sich bei dem eingesetzten Propan um frisches oder auch recyceltes Propan handeln, welches gegebenenfalls noch weitere, den Dehydrierungsprozess nicht wesentlich beeinflussende Nebenprodukte enthält.

Auch kann die Verfahrensführung der Propandehydrierung kontinuierlich sowie diskontinuierlich erfolgen.

Im Rahmen der Propandehydrierung in Schritt (i) des alternativen Verfahrens weist der aus diesem Schritt hervorgehende Teilstrom T (0) wenigstens Propen, Propan und Wasserstoff auf. Weiterhin kann T(0) als Nebenprodukte noch Gase, ausgewählt aus der Gruppe, bestehend aus N₂, H₂O, Methan, Ethan, Ethylen, CO und CO₂, einzeln oder als Gemisch von zwei oder mehr aus dieser Gruppe aufweisen.

Im Rahmen des erfindungsgemäßen Verfahrens liegt das Verhältnis von Propan zu Propen in Teilstrom T (0) im Bereich von 0,1 bis 10, bevorzugt 0,5 bis 5, besonders bevorzugt 1,0 bis 2,0. Das Verhältnis von Wasserstoff zu Propen in Teilstrom T (0) liegt im Bereich von 0 bis 1,5, bevorzugt 0,3 bis 1,3 besonders bevorzugt bei etwa 1,1.
Bei einer Fahrweise mit Sauerstoff als Co-Feed, d.h. mit zusätzlich zugeführtem Sauerstoff bzw. Kreisgas aus Schritt (iii), der Wasserstoffperoxidsynthese (T (6) bzw. T (6a)), liegt das Verhältnis von Wasserstoff zu Propen bevorzugt bei 0,4 bis 2,0.

Grundsätzlich wird Teilstrom T (0) ganz oder teilweise über geeignete, dem Fachmann bekannte Mittel, beispielsweise Leitungsvorrichtungen in Form von Rohren, in Schritt (ii) bzw. im erfindungsgemäßen erweiterten integrierten Verfahren zur Synthese von Propylenoxid in Schritt (b) überführt.

Weiterhin ist es auch möglich, dass Teilstrom T (0) in einem sich an Schritt (i) bzw. Schritt (a) anschließenden Zwischenschritt einer Separationseinrichtung zugeführt wird. In dieser können die gegebenenfalls im Rahmen der Propandehydrierung angefallenen Nebenprodukte, welche T (0) gegebenenfalls aufweist, abgetrennt werden.

Schritt (ii) bzw. Schritt (b) umfasst die Auftrennung des Teilstroms T (0) unter Erhalt wenigstens eines gasförmigen wasserstoffreichen Teilstroms T (2) und eines flüssigen Teilstroms T (1), welcher wenigstens Propen und Propan aufweist.

Die Auftrennung des Teilstroms T (0) kann im Rahmen der Erfindung grundsätzlich über alle dem Fachmann bekannten und für den vorliegenden Fall technisch möglichen Verfahrensweisen unter Einsatz der jeweilig geeigneten Vorrichtungen vorgenommen werden. Beispielsweise kann die Auftrennung mittels der in DE 100 28 582.1 beschriebenen Vorrichtung durchgeführt werden.

So kann die Abtrennung von T (1) aus Teilstrom T (0) über Schritt (ii) bzw. Schritt (b) so vorgenommen werden, dass der vorzugsweise abgekühlte Teilstrom T (0) mit einem vorzugsweise hydrophoben organischen Lösungsmittel in Kontakt gebracht wird, in welchem sich die in T (1) wenigstens enthaltenen Bestandteile Propan und Propen bevorzugt absorbieren lassen.

Durch nachfolgende Desorption, Rektifikation und/oder möglicherweise auch Strippung mit einem sich inert verhaltenden Gas und/oder einem Sauerstoff enthaltendem Gas, im Rahmen der Erfindung bevorzugt jedoch molekularem Sauerstoff, werden wenigstens die in T (1) vorhandenen Bestandteile Propan und Propen zurückgewonnen.

Der wenigstens Wasserstoff aufweisende Teilstrom T (2) stellt das Abgas der Absorption dar. Die Absorption kann dabei sowohl in Kolonnen als auch Rotationsabsorbem vorgenommen werden. Dabei kann im Gleichstrom oder im Gegenstrom gearbeitet werden. Geeignete Absorptionskolonnen sind z.B. Bodenkolonnen, Kolonnen mit strukturierten Packungen und Füllkörperkolonnen. Selbstverständlich kommen aber auch Riesel- und Sprühtürme, Granitblockabsorber, Oberflächenabsorber wie Dickschicht- und Dünnschichtabsorber sowie Rotationskolonnen, Tellerwäscher, Kreuzschleierwäscher und Rotationswäscher in Frage.

Grundsätzlich können alle dem Fachmann für diesen Zweck geeignet erscheinenden Absorptionsmittel eingesetzt werden. Vorzugsweise werden im Rahmen der vorliegenden Erfindung relativ unpolare organische Lösungsmittel, welche vorzugsweise keine nach außen wirkenden polaren Gruppen erhalten, z.B. aliphatische (z.B. C₈- bis C₁₈-Alkane), aber auch aromatische Kohlenwasserstoffe, z.B. Mittelölfraktionen aus der Paraffindestillation, oder auch Ether mit sperrigen Gruppen, angeordnet am Sauerstoffatom, eingesetzt. Geeignet sind auch Gemische aus zwei oder mehr der aufgeführten Lösungsmittel. Weitere als Absorptionsmittel im Rahmen der Erfindung einsetzbare Lösungsmittel oder Lösungsmittelgemische sind in der DE 100 28 582.1 aufgeführt.

Ein im Rahmen der vorliegenden Erfindung bevorzugt als Absorptionsmittel eingesetztes Lösungsmittelgemisch weist Diphenyl und Diphenylether, bevorzugt in der azeotropen Zusammensetzung, insbesondere aus etwa 25 Gew.-% Diphenyl (Biphenyl) und etwa 75 Gew.% Diphenylether (Diphyl® ) auf.

Im Rahmen der vorliegenden Erfindung wird Teilstrom T (0) jedoch bevorzugt durch Kondensation in die Teilströme T (1) und T (2) aufgetrennt.

So können im Rahmen des erfindungsgemäßen Verfahrens beispielsweise durch den Einsatz von Wärmetauschern, beispielsweise Oberflächenkondensatoren oder Kondensatoren mit direkter oder indirekter Luftkühlung, die C₃-Komponenten des Teilstroms T (0) ganz oder teilweise kondensiert werden.

Bevorzugt werden im Rahmen der Erfindung ein oder mehrere Rohrbündel-Wärmetauscher eingesetzt, wobei die Kühlung innerhalb der Wärmetauscher entweder mit Luft, Wasser oder einem anderen geeigneten Medium vorgenommen werden kann.

So kann der Teilstrom T (0) innerhalb Schritt (ii) bzw. Schritt (b) ganz oder teilweise kondensiert werden. Bevorzugt ist die weitgehend vollständige Kondensation der in Teilstrom T (0) enthaltenen C₃-Komponenten wie Propen und Propan.

Im Rahmen der Erfindung werden in Schritt (b) durch die oben erwähnten Auftrennungsmethoden mehr als 90 %, bevorzugt mehr als 95 %, besonders bevorzugt mehr als 99 % der in T (0) enthaltenen C₃-Komponenten als Teilstrom T (1) abgetrennt.

Teilstrom T (1), welcher wenigstens die C₃-Komponenten Propen und Propan aufweist, wird über geeignete, dem Fachmann bekannte Leitungsvorrichtungen. Schritt (iv) oder Schritt (c) zugeführt.

Die verbleibende Gasphase, Teilstrom T (2), umfasst neben Wasserstoff als Hauptkomponente gegebenenfalls noch einen variablen Anteil an C₃-Komponenten und gegebenenfalls noch weitere gasförmige, leichtsiedende Komponenten. Der Anteil an in T (2) vorhandenen C₃-Komponenten kann über die Bedingungen in Schritt (ii) bzw. Schritt (b) gesteuert werden. Die in Rede stehende Gasphase wird als Teilstrom T (2) über die dem Fachmann bekannte Leitungsvorrichtungen in Schritt (iii) überführt. Im Rahmen des erfindungsgemäßen erweiterten Verfahrens zur Synthese von Propylenoxid wird T (2) mit einem Teilstrom T (5a) vereinigt und über dem Fachmann bekannte Leitungsvorrichtungen in Schritt (e) überführt.

Im Rahmen des alternativen integrierten Verfahrens zur Synthese von Propylenoxid kann der gasförmige Teilstrom T (2) aus Schritt (ii) ein Verhältnis von Wasserstoff zu C₃-Komponenten in einem Verhältnis im Bereich von mindestens 90 bis 95 zu 10 bis 5, bevorzugt von 99 zu 1 und besonders bevorzugt von mindestens 99,9 zu 0,1 aufweisen.

Der flüssige Teilstrom T (1), welcher aus Schritt (ii) bzw. Schritt (b) hervorgeht und wenigstens Propen und Propan aufweist, wird in einem weiteren Schritt (iv) bzw. Schritt (c) unter Erhalt wenigstens eines propanreichen Teilstroms T (5) und wenigstens eines propenreichen Teilstroms T (3) aufgetrennt.

Die Auftrennung des Teilstroms T (1) kann durch alle dem Fachmann für diesen Zweck bekannten Verfahren erfolgen, bevorzugt jedoch durch thermische Verfahren wie Destillation und/oder Rektifikation.

Grundsätzlich können dabei alle für die Auftrennung des Teilstroms T (1) in einen propanreichen und einen propenreichen Teilstrom geeigneten Destillationsverfahren eingesetzt werden.

Die im Rahmen der Erfindung zu diesem Zweck eingesetzte Auftrennungseinheit weist grundsätzlich alle dem Fachmann bekannten Bestandteile auf, welche notwendig sind, um Stoffgemische durch Destillation in wenigstens eine propanreiche und eine propenreiche Fraktion aufzutrennen. Bevorzugt wird jedoch im Rahmen der vorliegenden Erfindung der Teilstrom T (1) durch Rektifikation in einen propanreichen und einen propenreichen Teilstrom aufgetrennt. Grundsätzlich erfolgt im Rahmen dieses Verfahrens die Anreicherung bzw. Trennung des Flüssigkeitsgemisches durch Stoffaustausch zwischen im Gegenstrom strömenden Gemischdampf und siedender Gemischflüssigkeit. Durchgeführt wird die Rektifikation in einer oder mehreren Rektifikationssäulen, welche im wesentlichen aus rohrartigen Trennsäulen sowie Verdampfern und einem Kondensator am oberen Ende (Kopf) der jeweiligen Säule besteht.

Im Rahmen des vorliegenden erfindungsgemäßen Verfahrens werden dabei mehr als 80 %, bevorzugt mindestens 90 %, besonders bevorzugt mindestens 95 % des sich in T(1) befindlichen Propens im Rahmen des Schritts (c) mit dem Teilstrom T (3) abgetrennt. Dieser propenreiche Teilstrom T (3) wird dem weiteren Schritt (g) über geeignete Leitungselemente zugeführt.

Der aus Schritt (iv) hervorgehende propanreiche Teilstrom T (5) kann wieder in Schritt (i) zurückgeführt werden.

Weiterhin ist es möglich, dass der propanreiche Teilstrom T (5) vor der Zuführung in den Schritt (i) durch weitere, dem Fachmann bekannte Verfahren aufbereitet wird, wobei beispielsweise das sich in ihm befindliche Propan vor der Zuführung in Schritt (i) angereichert wird.

Im Rahmen des erweiterten integrierten Verfahrens zur Synthese von Propylenoxid wird der aus Schritt (c) hervorgehende propanreiche Teilstrom T (5) über geeignete Leitungsvorrichtungen in Schritt (d) überführt.

Im Rahmen von Schritt (d) wird der Teilstrom T (5) in wenigstens die Teilströme T (5a) und T (5b) aufgetrennt. Im Rahmen einer bevorzugten Ausführungsform der vorliegenden Erfindung wird der Teilstrom T (5) vor der Auftrennung gereinigt, um die in ihm neben Propan gegebenenfalls enthaltenen Nebenprodukte abzutrennen. Diese Reinigung kann über alle dem Fachmann für diesen Zweck bekannten Verfahren, z.B. Destillations- oder Absorptionsverfahren, vorgenommen werden.

Die Auftrennung des Teilstroms T (5) kann über alle dem Fachmann für diesen Zweck zur Verfügung stehenden Verfahren, beispielsweise durch die Auftrennung mittels eines Mehrwegeventils erfolgen. Auch die Auftrennung unter gleichzeitiger Aufreinigung, beispielsweise mittels Destillation, ist möglich.

Grundsätzlich wird T (5) im Rahmen von Schritt (d) in zwei, gleiche oder unterschiedliche Mengen Propan aufweisende Teilströme T (5a) und T (5b) aufgetrennt.

Teilstrom T (5b) wird im Rahmen der vorliegenden Erfindung über dem Fachmann bekannte Leitungsvorrichtungen in Schritt (a) zurückgeführt.

Teilstrom T (5a) kann gegebenenfalls erneut gereinigt werden und wird sodann mit dem aus Schritt (b) hervorgehenden, Wasserstoff aufweisenden Teilstrom T (2) vereint und in Schritt (e) überführt.

Schritt (iii) des alternativen Verfahrens bzw. Schritt (e) des erfindungsgemäßen Verfahrens umfasst die Synthese von Wasserstoffperoxid.

Dabei wird im Rahmen von Schritt (iii) unter Einsatz des Wasserstoff aufweisenden Teilstroms T (2) und unter Zufuhr von Sauerstoff (X in Fig. 1), beispielsweise in Form von Luft umgesetzt, wobei ein wasserstoffperoxidreicher Teilstrom T (4) und ein gasförmiger Teilstrom T (6) erhalten werden.

Im Rahmen des erweiterten integrierten Verfahrens zur Synthese von Propylenoxid wird im Rahmen von Schritt (e) der Propan enthaltende Teilstrom T (5a), welcher mit dem Wasserstoff aufweisenden Teilstrom T (2) vereint wurde, unter Zufuhr eines Sauerstoff enthaltenden Gases, z.B. Luft oder von molekularem Sauerstoff (X in Fig. 2), umgesetzt, wobei ein wasserstoffperoxidreicher Teilstrom T (4) und ein gasförmiger Teilstrom T (6a) erhalten werden.

In einer bevorzugten Ausführungsform des erweiterten integrierten Verfahrens wird Sauerstoff an Stelle von Luft zugeführt. Somit wird die Anreicherung und die damit verbundene Ausschleusung eventuell störender Gase vermieden.

Gegebenenfalls kann über eine geeignete Vorrichtung neben Sauerstoff bzw. Luft auch Wasserstoff in Schritt (iii) bzw. Schritt (e) zudosiert werden (Z in Fig. 1 und Fig. 2).

Grundsätzlich können im Rahmen des Schritts (iii) bzw. Schritts (e) alle dem Fachmann bekannten Verfahren zur Wasserstoffperoxidsynthese eingesetzt werden.

In einer bevorzugten Ausführungsform des erfindungsgemäßen Verfahren wird Wasserstoffperoxid durch die Direktsynthese aus den Elementen hergestellt. Im Rahmen der vorliegenden Erfindung können alle dem Fachmann bekannten Verfahren zur Direktsynthese von Wasserstoffperoxid aus den Elementen eingesetzt werden.

Im Rahmen des erweiterten integrierten Verfahrens zur Synthese von Propylenoxid bietet der Einsatz der vereinigten Teilströme T (2) und T (5a) im Rahmen des Schritts (e) den Vorteil, dass damit eine Absenkung der Sauerstoffmenge im Reaktionsgemisch der Wasserstoffperoxidsynthese unter die untere Explosionsgrenze (4 % H₂ zu 96 % O₂) erreicht wird, wodurch die Sicherheit der Verfahrensführung erhöht wird. Das durch T (5a) zugesetzte Propan stellt in dieser Ausführungsform der Erfindung somit ein sicheres Gaspolster für die Wasserstoffperoxiddirektsynthese dar.

Einen weiteren damit im Zusammenhang stehenden Vorteil stellt die dadurch ermöglichte Erhöhung der Wasserstoffkonzentration auf ein Verhältnis von Sauerstoff zu Wasserstoff von 1 zu 1 oder höher dar. Da die Raum-Zeit-Ausbeute der Wasserstoffperoxiddirektsynthese proportional zur Wasserstoffkonzentration ansteigt, ist es im Rahmen dieser Ausführungsform der Erfindung möglich, den Reaktor räumlich zu verkleinern und somit auch die Betriebskosten zu senken.

Im Rahmen des Schritts (e) des erfindungsgemäßen erweiterten Verfahrens zur Synthese von Propylenoxid wird die Menge an Sauerstoff und Wasserstoff im Feed so dosiert, dass in dem aus diesem Schritt hervorgehenden Teilstrom T (6a) ein für dessen Verwertung im Rahmen der Propandehydrierung des Schrittes (a) optimales Verhältnis vorliegt.

Demgemäß wird im Rahmen des erfindungsgemäßen erweiterten Verfahrens zur Synthese von Propylenoxid der aus Schritt (e) hervorgehende Teilstrom T (6a) in Schritt (a) zurückgeführt und ermöglicht dadurch die autotherme Fahrweise der Propandirektsynthese, wie oben beschrieben.

Durch diese Verfahrensweise ist es somit im Rahmen der Wasserstoffperoxiddirektsynthese möglich, auf Kreisgas zu verzichten.

Beispiele für mögliche Verfahren zur Wasserstoffperoxidherstellung im Rahmen der vorliegenden Erfindung ist die in US 4,009,252 offenbarte Vorgehensweise, bei welcher Wasserstoffperoxid aus Wasserstoff und Sauerstoff an palladiumhaltigen Katalysatoren gebildet wird. Diese Umsetzung erfolgt nach dem Batch-Verfahren. Auch die WO 92/04277 beschreibt eine im Rahmen der Erfindung einsetzbare Vorgehensweise zur Umsetzung von Wasserstoff mit Sauerstoff in einem mit wässriger Katalysatorsuspension gefüllten Rohrreaktor zu Wasserstoffperoxid. Eine weitere Möglichkeit zur Herstellung von Wasserstoffperoxid im Rahmen der vorliegenden Erfindung stellt das in US-A 5,500,202 sowie EP-A 0 579 109 beschriebene kontinuierliche Verfahren zur Herstellung von Wasserstoffperoxid durch Umsetzung von H₂O₂-Gasmischungen an einem stationären, pulverförmigen Katalysator in einem Rieselbett-Reaktor dar.

Ein weiteres im Stand der Technik bekanntes und im Rahmen der vorliegenden Erfindung einsetzbares Verfahren zur Herstellung von Wasserstoffperoxid wird in US-A 4,336,238 und US-A 4,336,239 beschrieben. In diesem Verfahren erfolgt die Umsetzung von Wasserstoff und Sauerstoff zu Wasserstoffperoxid an palladiumhaltigen Katalysatoren in organischen Lösungsmitteln oder Lösungsmittelmischungen, welche gegebenenfalls auch Wasser enthalten können. Die US-A 4,389,390 beschreibt ein ähnliches Verfahren, wobei der Katalysator, der sich vom Träger gelöst hat, durch Aktivkohlefilter zurückgewonnen wird.

Besonders bevorzugt wird im Rahmen der vorliegenden Erfindung jedoch das von der Anmelderin selbst entwickelte und in EP-A 0 946 409 beschriebene Verfahren zur Herstellung von Wasserstoffperoxid-Lösungen verwendet. Mit Hilfe dieses Verfahrens gelingt die sichere Herstellung von Wasserstoffperoxid-Lösungen mit einem Wasserstoffperoxid-Gehalt von wenigstens 2,5 Gew.-%. Dabei wird Wasserstoff und Sauerstoff kontinuierlich an Katalysatoren umgesetzt, wobei die Katalysatoren als aktive Komponente Palladium enthalten und die Umsetzung in Wasser und/oder C₁-C₃-Alkanolen als Reaktionsmedium an Katalysatorformkörpern erfolgt. Bei den Katalysatorformkörpern handelt es sich dabei bevorzugt um geordnete Katalysatorpackungen (Monolithe) und/oder -schüttungen oder um aus Gewebe beispielsweise Gewebe aus Metall aufgebaute Formkörper. Im Rahmen dieses bevorzugten Verfahrens ist es möglich, Sauerstoff in Form von Luft zu verwenden.

Die Durchführung der Reaktion im Rahmen dieses Verfahrens erfolgt in der Regel bei geflutetem Reaktor. Als Reaktionsmedium dienen Wasser und/oder C₁-C₃-Alkanole, vorzugsweise Wasser und/oder Methanol. Das Reaktionsgas, das neben Wasserstoff und Sauerstoff auch noch inerte Gase wie Stickstoff oder Edelgase enthalten kann, weist in der Regel ein O₂:H₂-Verhältnis im Bereich von 1 : 100 bis 100 : 1 auf. Es ist möglich, das Reaktionsgas im Kreis zu führen. Reaktionsgas und Reaktionsmedium können im Gleichstrom oder im Gegenstrom zueinander, vorzugsweise im Gleichstrom geführt werden, wobei die flüssige Phase die kontinuierliche und das Reaktionsgas die diskontinuierliche Phase bilden. Bevorzugt wird ein vertikaler Reaktoraufbau (stehender Reaktor), in welchem Reaktionsgas und Reaktionsmedium vorzugsweise im Gleichstrom von unten nach oben durch den Reaktor geführt werden. Hierbei kann Wasserstoff über eine oder mehrere Zwischeneinspeisungen stromabwärts vom Einspeisungspunkt des Sauerstoffs oder der Luft dem Reaktor zugeführt werden. Am oberen Ende des Reaktors kann der zweiphasige Reaktoraustrag entnommen und in einem dafür geeigneten Trenngefäß in einen wasserstoffperoxidreichen Teilstrom T (4) und einen gasförmigen Teilstrom T (6) bzw. T (6a) aufgetrennt werden.

Im Rahmen das Verfahrens wird die Wasserstoffperoxidsynthese in Schritt (iii) derart durchgeführt, dass auch bei Verwendung von Wasserstoff/Sauerstoff-Mischungen außerhalb des Explosiv-Bereiches (O₂:H₂ > 20:1), Wasserstoffperoxid-Lösungen mit einem Wasserstoffperoxid-Gehalt oberhalb 2,5 Gew.-% erhalten werden.

Der wasserstoffperoxidreiche Teilstrom T (4) weist wenigstens Wasserstoffperoxid und Wasser auf. Gegebenenfalls weist Teilstrom T (4) zusätzlich noch Halogenide, Säuren, Alkohole und weitere organische Komponenten sowie Sensitizer und Promotoren der Wasserstoffperoxidsynthese, z.B. CO auf.

Gegebenenfalls kann T (4) über dem Fachmann bekannte Verfahren weiter aufgearbeitet werden.

Demgemäß betrifft die Erfindung auch ein Verfahren der oben beschriebenen Art, wobei Teilstrom T (4) wenigstens Wasserstoffperoxid und Wasser aufweist.

Die Gasphase T(6) kann über einen geeigneten Verdichter oder aber auch direkt ganz oder teilweise in einem weiteren Schritt (vi) zur Energiegewinnung verbrannt und die Energie innerhalb des Schrittes (i) verwertet werden.

Besonders bevorzugt wird diese Vorgehensweise gewählt, wenn der aus Schritt (iii) hervorgehende Teilstrom T (6) weniger als 4 % Wasserstoff in Sauerstoff, bzw. weniger als 4 % Sauerstoff in Wasserstoff aufweist.

Die so im Rahmen von Schritt (vi) gewonnene Energie kann innerhalb des Schrittes (i) zur Beheizung der im Rahmen der Propandehydrierung verwendeten Einrichtungen und/oder zur Regeneration des wenigstens einen, im Rahmen der Propandehydrierung eingesetzten Katalysators dienen.

Ebenso ist es möglich die Verwertung der Energie innerhalb des Schrittes (i) durch folgende Schritte einzeln oder in Kombination miteinander vorzunehmen:
(aa) Beheizung der im Rahmen der Propandehydrierung verwendeten Einrichtungen;
(bb) Regeneration des wenigstens einen, im Rahmen der Propandehydrierung eingesetzten Katalysators.

Teilstrom T (6) kann grundsätzlich ganz oder teilweise in Schritt (iii) zurückgeführt werden.

Um einer im Fall der vollständigen Rückführung von T (6) gegebenenfalls auftretenden Aufpegelung von inerten Verbindungen entgegenzuwirken, wird zeitweise oder über den gesamten Verlauf des Verfahrens lediglich ein Teilstrom von T (6) zur Verwertung in Schritt (i) überführt. Diese Vorgehensweise wird bevorzugt immer dann gewählt, wenn der gasförmige Teilstrom T (6) mehr als 0,5 %, bevorzugt mehr als 0,7 %, besonders bevorzugt mehr als 1 % eines Gemisches aus Wasserstoff und Sauerstoff aufweist.

Im Rahmen des alternativen Verfahrens zur Synthese von Propylenoxid kann der aus Schritt (iii) hervorgehende gasförmige Teilstrom T (6) ganz oder teilweise in Schritt (i) zurückgeführt werden. Der Vorteil dieser Vorgehensweise ist, dass der gasförmige Teilstrom T (6), welcher im Rahmen dieser Ausführungsform neben C₃₋Resten ein Verhältnis von H₂:O₂ im Bereich von 1 : 100 bis 100 : 1 aufweist, zur Regeneration des in Schritt (i) eingesetzten Katalysators zur Propandehydrierung verwendet werden kann. Die Regenerierung erfolgt grundsätzlich durch vollständiges oder teilweises Abbrennen der sich auf der Katalysatoroberfläche abgeschiedenen organischen Komponenten.

Der aus Schritt (iv) bzw. Schritt (c) hervorgehende Teilstrom T (3) wird in Schritt (v) mit dem Teilstrom T (4), welcher aus Schritt (ii) bzw. Schritt (e) hervorgeht, in einem weiteren Schritt (v) bzw. Schritt (g) unter Erhalt von Propylenoxid umgesetzt.

Die Gasphase T (6a) aus Schritt (e) des erfindungsgemäßen Verfahrens kann aber auch direkt in einem weiteren Schritt (f) in den Schritt (a) zurückgeführt werden. In diesem Fall besteht der Teilstrom T (6a) aus den Komponenten Propan, Sauerstoff und Wasserstoff in einem molaren Mischungsverhältnis C₃:O₂:H₂ von 1:0,01-1:0-2, bevorzugt in einem molaren Mischungsverhältnis C₃:O₂:H₂ von 1:0,03-0,3:0-0,6 und besonders bevorzugt in einem molaren Mischungsverhältnis C₃:O₂:H₂ von 1:0,04-0,2:0-0,4.

Die Umsetzung des propanreichen Teilstroms T (3) mit dem wasserstoffperoxidreichen Teilstrom T (4) unter Erhalt von Propylenoxid kann über alle dem Fachmann bekannten Verfahren erfolgen.

Im Rahmen der vorliegenden Erfindung handelt es sich bei der Umsetzung in Schritt (g) bevorzugt um die Epoxidation des Propens aus Teilstrom T (3) mit Wasserstoffperoxid aus Teilstrom T (4) in Gegenwart eines Katalysators zu Propylenoxid.

Mögliche Verfahrensführungen im Rahmen der in Rede stehenden Epoxidation sowie bevorzugt eingesetzte Epoxidationskatalysatoren sind u.a. in DE 101 35 296.4, DE 101 05 528.5, DE 100 32 884.9, DE 101 554 70.2, DE 101 37 543.3 sowie DE 101 35 296.4 beschrieben.

Im Rahmen der Erfindung besonders bevorzugt eingesetzte Epoxidationskatalysatoren sind Ti-Zeolithe mit MFI-, MEL- oder MFI/MEL-Mischstrukturen, als TS-1, TS-2, TS-3 bezeichnete, Ti enthaltende Zeolithkatalysatoren sowie Ti-Zeolithe mit einer zu β-Zeolith i-somorphen Gerüststruktur. Weitere Details bezüglich der einsetzbaren Katalysatoren, insbesondere Zeolithe, sind u. a. der DE 100 10 139.2, der DE 197 23 950.1 sowie der DE 102 32 406.9 und dem darin jeweils zitierten Stand der Technik zu entnehmen.

Demgemäß betrifft die vorliegende Erfindung auch ein Verfahren nach oben beschriebener Art, wobei es sich bei der Umsetzung in Schritt (v) um die Epoxidation des Propens aus Teilstrom T (3) mit Wasserstoffperoxid aus Teilstrom T (4) in Gegenwart eines Katalysators zu Propylenoxid handelt.

Im Rahmen der vorliegenden Erfindung beträgt die Umsetzungsrate zu Propylenoxid mindestens 80 %, bevorzugt mindestens 85 %, besonders bevorzugt mindestens 95 %.

Das Propylenoxid kann von dem bei der Umsetzung in Schritt (v) bzw. Schritt (g) entstandenen Gemisch durch alle dem Fachmann bekannten Verfahren abgetrennt und gegebenenfalls weiter aufgearbeitet werden. Die im Rahmen der vorliegenden Erfindung bevorzugten Abtrennungs- und Aufarbeitungsverfahren werden in DE 198 35 907.1 sowie DE 100 01 401.1 beschrieben.

Das neben Propylenoxid in Schritt (v) bzw. Schritt (g) entstehende Gemisch kann als Teilstrom T (7), welcher wenigstens Propan und Propen aufweist, ganz oder teilweise in Schritt (i) bzw. Schritt (a) zurückgeführt werden.

Demgemäß betrifft die vorliegende Erfindung auch ein Verfahren der oben beschriebenen Art, wobei aus Schritt (g) ein Teilstrom T (7) hervorgeht, welcher wenigstens Propan und Propen aufweist und bei einem Verhältnis von Propan zu Propen kleiner 1 gegebenenfalls nach einem weiteren Aufarbeitungsschritt ganz oder teilweise in Schritt (a) oder direkt in Schritt (c) zurückgeführt wird.

Der in Rede stehende Aufarbeitungsschritt kann über dem Fachmann bekannten Verfahren, beispielsweise Destillation, Rektifikation bzw. Membrantrennung erfolgen.

Die nachfolgenden Tabellen 1 und 2 illustrieren mögliche Ausführungsformen des alternativen und des erfindungsgemäßen Verfahrens, wobei sich Tabelle 1 auf das alternative integrierte Verfahren, das schematisch in Fig.1 dargestellt ist, und Tabelle 2 auf das erweiterte integrierte Verfahren, das schematisch in Fig.2 dargestellt ist, beziehen.

### Bezugszeichenliste

Fig. 1
   - Schritt (i): Propandehydrierung
   - Schritt (ii): Kondensation
   - Schritt (iii): Wasserstoffperoxidsynthese
   - Schritt (iv): Auftrennung
   - Schritt (v): Propylenoxid-Synthese
   - Schritt (vi): Verbrennung
   - T (0), T (1), T (2), T (3), T (4), T (5), T (6), T (7): Teilströme
   - X: Luft oder Sauerstoff
   - Y: Propylenoxid
   - Z: Wasserstoff
Fig.2
   - Schritt (a): Propandehydrierung
   - Schritt (b): Kondensation
   - Schritt (c): Auftrennung
   - Schritt (d): Auftrennung
   - Schritt (e): Wasserstoffperoxidsynthese
   - Schritt (f): Rückführung
   - Schritt (g): Propylenoxid-Synthese
   - T (0), T (1), T (2), T (3), T (4), T (5), T (5a), T (5b), T (6a), T (7): Teilströme
   - X: Sauerstoff
   - Y: Propylenoxid
   - Z: Wasserstoff

## Patentansprüche

1. Integriertes Verfahren zur Synthese von Propylenoxid, **dadurch gekennzeichnet, dass** das Verfahren wenigstens die folgenden Schritte umfasst:
(a) Propandehydrierung unter Erhalt eines Teilstroms T (0), welcher wenigstens Propan, Propen und Wasserstoff aufweist;
(b) Auftrennung des Teilstroms T (0) unter Erhalt wenigstens eines gasförmigen wasserstoffreichen Teilstroms T (2) und eines Teilstroms T (1), welcher wenigstens Propen und Propan aufweist;
(c) Auftrennung des Teilstroms T (1) unter Erhalt wenigstens eines propanreichen Teilstroms T (5) und wenigstens eines propenreichen Teilstrom T (3);
(d) Auftrennen des Teilstroms T (5) in wenigstens die Teilströme T (5a) und T (5b);
(e) Wasserstoffperoxidsynthese unter Einsatz des Teilstroms T (2) welcher wenigstens mit dem Teilstrom T (5a) vereint wird, wobei ein wasserstoffperoxidreicher Teilstrom T (4) und ein gasförmiger Teilstrom T (6a) erhalten werden;
(f) zumindest teilweise Rückführung des Teilstroms T (6a) in Schritt (a);
(g) Umsetzung des wenigstens einen Teilstroms T (3) mit Teilstrom T (4) unter Erhalt von Propylenoxid.

2. Verfahren nach Anspruch 1, **dadurch gekennzeichnet, dass** der propanreiche Teilstrom T (5b) Schritt (a) zugeführt wird.

3. Verfahren nach Anspruch 1 oder 2, **dadurch gekennzeichnet, dass** Teilstrom T (4) wenigstens Wasserstoffperoxid und Wasser aufweist.

4. Verfahren nach einem der Ansprüche 1 bis 3, **dadurch gekennzeichnet, dass** es sich bei der Umsetzung in Schritt (g) um die Epoxidation des Propens aus Teilstrom T (3) mit Wasserstoffperoxid aus Teilstrom T (4) in Gegenwart eines Katalysators zu Propylenoxid handelt.

5. Verfahren nach einem der Ansprüche 1 bis 4, **dadurch gekennzeichnet, dass** aus Schritt (g) ein Teilstrom T (7) hervorgeht, welcher wenigstens Propan und/oder Propen aufweist und ganz oder teilweise in Schritt (a) zurückgeführt wird.

6. Verfahren nach einem der Ansprüche 1 bis 5, **dadurch gekennzeichnet, dass** aus Schritt (g) ein Teilstrom T (7) hervorgeht, welcher wenigstens Propan und Propen aufweist und bei einem Verhältnis von Propan zu Propen kleiner 1 gegebenenfalls nach einem weiteren Aufarbeitungsschritt ganz oder teilweise in Schritt (c) zurückgeführt wird.

## Claims

1. An integrated process for the synthesis of propylene oxide, which comprises at least the following steps:
(a) dehydrogenation of propane to give a substream T (0) comprising at least propane, propene and hydrogen;
(b) fractionation of the substream T (0) to give at least one gaseous hydrogen-rich substream T (2) and a substream T (1) comprising at least propene and propane;
(c) fractionation of the substream T (1) to give at least one propane-rich substream T (5) and at least one propene-rich substream T (3);
(d) separation of the substream T (5) into at least the substreams T (5a) and T (5b);
(e) synthesis of hydrogen peroxide using the substream T (2) which is combined with at least the substream T (5a), giving a substream T (4) which is rich in hydrogen peroxide and a gaseous substream T (6a);
(f) at least partial recirculation of the substream T (6a) to step (a);
(g) reaction of the substream or substreams T (3) with substream T (4) to give propylene oxide.

2. The process according to claim 1, wherein the propane-rich substream T (5b) is fed to step (a).

3. The process according to claim 1 or 2, wherein substream T (4) comprises at least hydrogen peroxide and water.

4. The process according to any of claims 1 to 3, wherein the reaction in step (g) is the epoxidation of the propene from substream T (3) by means of hydrogen peroxide from substream T (4) in the presence of a catalyst to give propylene oxide.

5. The process according to any of claims 1 to 4, wherein a substream T (7) comprising at least propane and/or propene is obtained from step (g) and is wholly or partly recirculated to step (a).

6. The process according to any of claims 1 to 5, wherein a substream T (7) comprising at least propane and propene and having a ratio of propane to propene of less than 1 is obtained from step (g) and is, if appropriate after a further work-up step, wholly or partly recirculated to step (c).

## Revendications

1. Procédé intégré pour la synthèse d'oxyde de propylène, **caractérisé en ce que** le procédé comprend les étapes suivantes :
(a) déshydrogénation de propane pour obtenir un courant partiel T(0) qui présente au moins du propane, du propène et de l'hydrogène;
(b) séparation du courant partiel T(0) pour obtenir au moins un courant partiel gazeux riche en hydrogène T(2) et un courant partiel T(1) qui présente au moins du propène et du propane;
(c) séparation du courant partiel T(1) pour obtenir au moins un courant partiel riche en propane T(5) et au moins un courant partiel riche en propène T(3);
(d) séparation du courant partiel T(5) en au moins les courants partiels T(5a) et T(5b);
(e) synthèse de peroxyde d'hydrogène en utilisant le courant partiel T(2) qui est au moins combiné avec le courant partiel T(5a) pour obtenir un courant partiel riche en peroxyde d'hydrogène T(4) et un courant partiel gazeux T(6a);
(f) recyclage au moins partiel du courant partiel T(6a) à l'étape (a); et
(g) réaction du au moins un courant partiel T(3) avec le courant partiel T(4) pour obtenir de l'oxyde de propylène.

2. Procédé selon la revendication 1, **caractérisé en ce que** le courant partiel riche en propane T(5b) est acheminé à l'étape (a).

3. Procédé selon la revendication 1 ou 2, **caractérisé en ce que** le courant partiel T(4) présente au moins du peroxyde d'hydrogène et de l'eau.

4. Procédé selon l'une quelconque des revendications 1 à 3, **caractérisé en ce que** la réaction à l'étape (g) consiste à époxyder le propène du courant partiel T(3) avec du peroxyde d'hydrogène du courant partiel T(4) en présence d'un catalyseur pour former de l'oxyde de propylène.

5. Procédé selon l'une quelconque des revendications 1 à 4, **caractérisé en ce qu'**il résulte de l'étape (g) un courant partiel T(7) qui présente au moins du propane et/ou du propène et qui est complètement ou partiellement recyclé à l'étape (a).

6. Procédé selon l'une quelconque des revendications 1 à 5, **caractérisé en ce qu'**il résulte de l'étape (g) un courant partiel T(7) qui présente au moins du propane et/ou du propène et qui, dans un rapport du propane au propène inférieur à 1, est éventuellement, après une autre étape de traitement, complètement ou partiellement recyclé à l'étape (c).
